# EUROPEAN PATENT APPLICATION

(11) **EP 4 600 651 A1**
(43) Date of publication of application: **13.08.2025**
(21) Application number: 25156098.3
(22) Date of filing: 05.02.2025
(51) Int. Cl.: G01N 33/68, G01N 33/564

(54) **METHOD FOR DIAGNOSING COELIAC DISEASE**

(30) Priority: 06.02.2024 IT 202400002407
(71) Applicant: Consiglio Nazionale Delle Ricerche - CNR, 00185 Roma (IT)
(72) Inventor: DEL POZZO, Giovanna, 00185 ROMA (IT); GIANFRANI, Carmela, 00185 ROMA (IT); PISAPIA, Laura, 00185 ROMA (IT)
(74) Representative: Delbarba, Andrea

(57) **Abstract**

The present invention relates to a method for diagnosing coeliac disease in an individual.

Furthermore, the present invention relates to a kit and the use thereof for diagnosing coeliac disease in an individual.

## Description

### FIELD OF THE INVENTION

The present invention relates to a method for diagnosing coeliac disease in an individual. In particular, said method comprises identifying and verifying the activation of antigen-specific T cells, of the CD4⁺ subtype, in coeliac disease.

Furthermore, the present invention relates to a kit and the use thereof for diagnosing coeliac disease in an individual.

### STATE OF THE ART

Coeliac disease is a permanent inflammatory disease of the intestine caused by the consumption of foods containing gluten.

In fact, in individuals with coeliac disease, the intake of a food containing gluten triggers an immune response that affects the small intestine and, in some cases, results in damage to the fundamental structures of the small intestine, in particular the intestinal villi, causing a flattening of the latter and consequently an inability to absorb nutrients.

At present, coeliac disease is diagnosed by measuring immunoglobulin (IgA) anti-transglutaminase (anti-tTG) and anti-endomysial (EMA) antibodies in serum. In the presence of seropositivity, adult individuals with suspected coeliac disease undergo an assessment of small intestine mucosal atrophy by means of a gastrointestinal endoscopic investigation (EGD), without a doubt an invasive practice.

According to the recent guidelines of the European Society for Paediatric Gastroenterology Hepatology and Nutrition (ESPGHAN, 2020), a diagnosis of coeliac disease can be made in individuals of paediatric age with a positive anti-tTG test alone and the presence of clear symptoms, without subjecting children to the invasive practice of digestive endoscopy. However, there are reports in the literature of various types of situations in which a diagnostic doubt persists and it becomes necessary to observe the damage of the mucosa by means of an EGD investigation. Antigen-specific T cells play a fundamental role in the immune response against infections and against self-antigens in autoimmune diseases. The most common techniques used to study antigen-specific CD4⁺ and CD8⁺ T cells are based on measuring the cytokines produced following antigenic stimulation. These methods are the ICS (intracellular cytokine staining) assay, which consists in an intracellular determination of the cytokines produced (in particular IFNγ) by staining with a specific antibody, or immunoenzymatic assays such as ELISA and ELISPOT. However, these techniques have some limitations, due to the laboriousness of the methods and the small amount and/or the type of cytokines detected, which can influence the sensitivity of the test.

In particular, ICS requires a long process of cell permeabilization, with a considerable consumption of reagents and a reduced sensitivity of the test. On the other hand, ELISA and ELISPOT do not provide information on the various lymphocyte subpopulations responsible for the immune response.

Furthermore, the ICS and ELISPOT assays only detect cells that produce cytokines above a certain threshold, though those that produce low levels of cytokines can also be biologically relevant. A further method for detecting activated T cells in blood is based on HLA multimer (tetramer or dextramer) technology. However, this technique has various limitations, including a high cost and the need to know the HLA genotype and sequences of the antigen peptides presented.

In reality, to date the diagnosis of coeliac disease has mainly been made through a determination of anti-TG antibodies and histological assessment of intestinal mucosal damage in biopsy samples.

However, there are many controversial cases in which a diagnosis cannot be made with certainty. In fact, in some individuals, the detection of autoantibodies and assessment of intestinal atrophy in biopsy samples do not enable an unambiguous interpretation.

It is necessary, therefore, to provide a method that enables a diagnosis of active coeliac disease to be made with certainty without relying on a histopathological analysis of intestinal mucosal tissue.

Furthermore, there is a felt need for a method which makes it possible, over time, to monitor remission of the disease following a gluten-free diet, without relying on invasive practices.

At present, the only effective therapy for treating coeliac disease is a gluten-free diet. This dietary regimen, though effective in eliminating the symptoms and restoring intestinal function, poses many restrictions, above all as regards compliance in teenage years. In view of this, it is fundamental to monitor the dietary pattern and, in general, the pattern of the disease.

### SUMMARY OF THE INVENTION

A **first** aspect of the present invention relates to an in vitro method for determining the presence of coeliac disease in an individual, comprising at least the following steps:
a) putting at least one sample of blood isolated from the individual in contact with at least one portion of gliadin,
b) detecting the presence of activated CD4⁺ T cells in said isolated sample,

wherein a higher amount of activated T cells detected in step b) compared to an amount of activated T cells detected in a sample isolated from an individual without coeliac disease indicates the presence of coeliac disease in the individual,
and wherein step b) comprises the detection of two activation markers expressed by said CD4⁺ T cells.

A second aspect of the present invention relates to a kit comprising at least one portion of gliadin as defined above and at least one molecule capable of specifically binding two T-cell activation markers: OX40 and CD137.

A third aspect of the present invention relates to the use of said kit for determining the presence of coeliac disease in an individual, preferably by means of the in vitro method according to the first aspect of the invention.

### BRIEF DESCRIPTION OF THE FIGURES

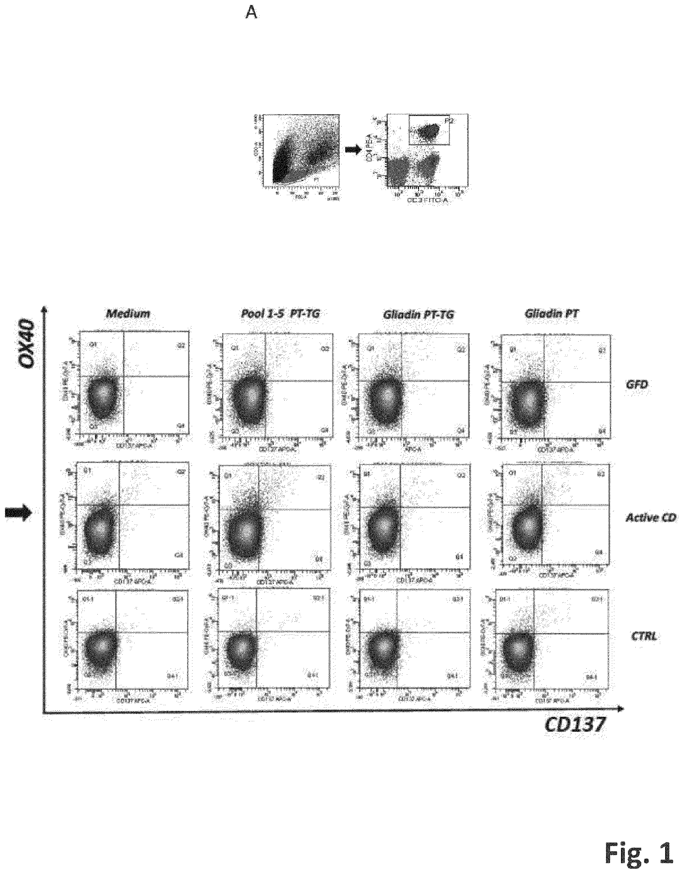

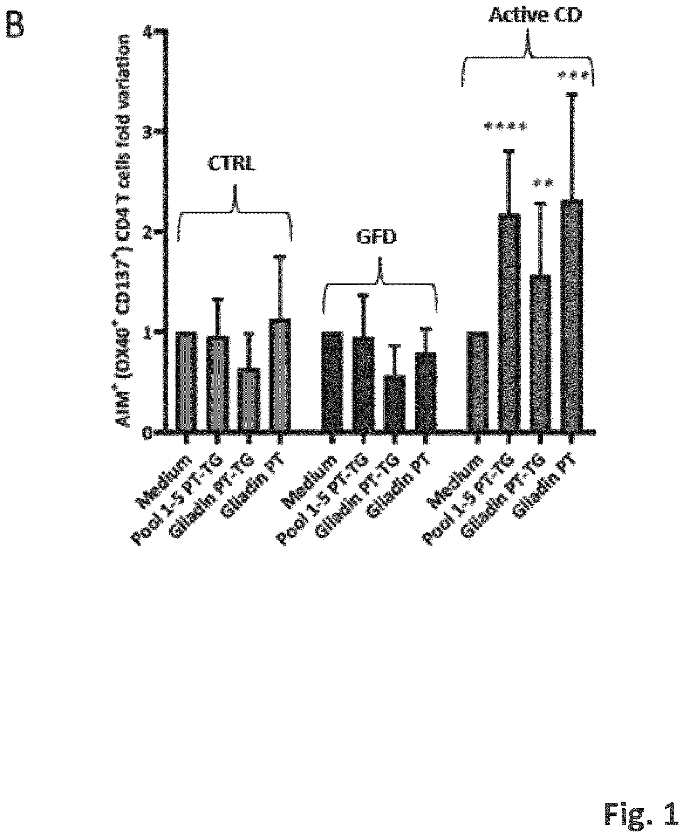

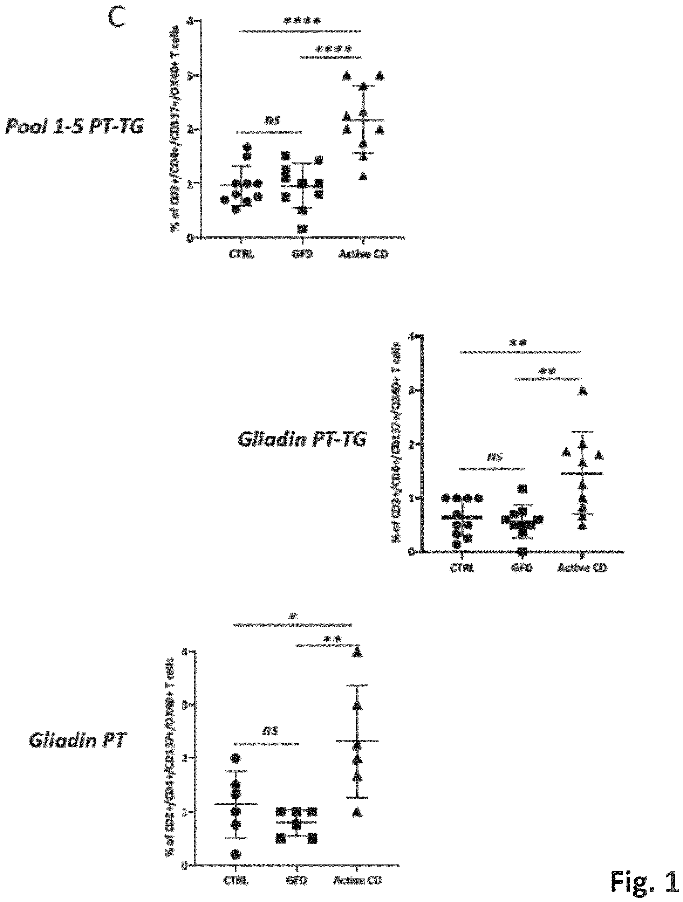

Figure 1 shows the results of the experiments described in the experimental section.

In particular, the activated CD4⁺ T cells (OX40⁺CD137⁺) are highlighted in samples of peripheral blood mononuclear cells (PBMCs) originating from three cohorts of subjects: healthy volunteers (CTRL, 10 subjects), patients with acute coeliac disease (CeD, 10 subjects) and patients in remission (GFD, 10 subjects). The cells were stimulated with the gliadin peptides presented by the HLA-DQ2.5 molecule having the sequences (SEQ: ID: NO.5) - (SEQ: ID: NO.9) (indicated in Figure 1 as "p1-p5"), with the peptic-tryptic digest of deamidated native gliadin (indicated in Figure 1 as "gliadin PT-TG"), and with the peptic-tryptic digest of native gliadin (indicated in Figure 1 as "gliadin PT").

Panel A: Representative cytofluorometric analysis.

Panel B: Mean value of CD3⁺/CD4⁺/OX40⁺/CD137⁺ cells detected for each cohort of subjects analysed, expressed as a variation compared to the negative control represented by the medium alone.

Panel C: distribution of cell values for each subject belonging to the three cohorts, after antigenic stimulation.

All data were normalised to the negative control. Student's t-test was unpaired, with a two-tailed distribution, ** p <0.01, *** p < 0.001; **** p < 0.0001.

### DEFINITIONS

In the context of the present description, the expression "activated T cells" is meant to indicate CD4+ T cells which express the two markers OX40 and CD137 on the cell surface.

In fact, the presence of such markers demonstrates that the T cells have come into contact with the antigen.

### DETAILED DESCRIPTION OF THE INVENTION

A **first** aspect of the present invention relates to an in vitro method for determining the presence of coeliac disease in an individual, comprising at least the following steps:
a) putting at least one sample isolated from the individual into contact with at least one portion of gliadin,
b) detecting the presence of activated CD4+ T cells in said isolated sample,

wherein a higher amount of activated T cells detected in step b) compared to an amount of activated T cells detected in a sample isolated from an individual without coeliac disease indicates the presence of coeliac disease in the individual,
and wherein step b) comprises the detection of two CD4+ T cell activation markers expressed by said CD4+ T cells.

In other words, the method of the present invention is a method for detecting the presence of T cells, i.e. activated CD4⁺ T cells, in a sample isolated from an individual in response to a stimulus. The CD4⁺ T cells present in the isolated sample are not naturally activated but are rather activated following and in response to stimulation with the protein or a part of it as defined in step a) and in the present application. The presence of activated CD4⁺ T cells in the isolated sample is determined thanks to the detection of the expression of two CD4⁺ T cell activation markers, on or by said T cells.

Therefore, the invention relates to a method for detecting the presence of CD4⁺ T cells which are reactive to the protein or to at least a portion of it as defined in step a) and in the present application. Preferably, said method allows for detecting, in an isolated blood sample, the presence of T cells which are reactive to at least a toxic and/or immunogenic portion of the protein as defined above, i.e. gliadin. The detection of the presence of reactive CD4⁺ T cells takes place thanks to the detection of the expression of two markers that identify the T cell population: CD3 and CD4.

In a particularly preferred embodiment, said sample isolated from a subject is a blood sample, in particular a peripheral blood sample, or another sample containing CD4⁺ T cells.

Preferably, the sample is used directly in the method of the present invention, or else it has been previously processed. The isolated sample is preferably processed with techniques known to the person skilled in the art, e.g. dilution and/or purification.

The blood sample is used to prepare peripheral blood mononuclear cells (PBMCs) which include CD4⁺ T and CD8⁺ T cells.

In particular, the peripheral blood mononuclear cells (PBMCs) comprise lymphocytes (T cells, B cells, NK cells) and monocytes.

In one embodiment, the sample is isolated from a symptomatic or asymptomatic individual with coeliac disease, or one suspected to be affected by coeliac disease.

In one embodiment, the individual is susceptible to coeliac disease, preferably genetically susceptible to coeliac disease.

In one embodiment, in step a) the at least one portion of gliadin is selected from SEQ ID NO.1, SEQ ID NO.2, SEQ ID NO.3 and SEQ ID NO.4, or has at least one protein with a sequence identity of at least 80%, preferably at least 90%, 95%, 96%, 97%, 98%, 99% with SEQ ID NOs.1-4.

Preferably, the at least one portion of gliadin comprises at least one epitope able to be recognised by the CD4⁺ T cells of the immune system of the individual and to activate an immune response in the individual. Preferably, the at least one portion of gliadin can be and/or is bound by the class II molecule HLA-DQ2.5.

Gliadin is a protein which is a component of gluten, particularly present in wheat flours (including spelt flours, and flours of other cereals of the genus *Triticum),* barley and rye.

Preferably, the at least one portion of gliadin comprises or consists of a sequence that is at least 80% identical with a sequence selected from SEQ: ID: NO.5, SEQ: ID: NO. 6, SEQ: ID: NO. 7, SEQ: ID: NO. 8, and SEQ: ID: NO. 9, shown in Table 1.

In one embodiment, step a) envisages putting the isolated sample into contact with at least two, or three, or four or five portions of gliadin. Preferably, said at least two, or three, or four or five portions comprise or consist of sequences that are at least 80% identical with the sequences selected from SEQ: ID: NO.5, SEQ: ID: NO. 6, SEQ: ID: NO. 7, SEQ: ID: NO. 8 and SEQ: ID: NO. 9.

Preferably, said at least one portion or said at least two, three, four or five portions of gliadin comprise or consist of sequences which are at least 90%, 91%, 92%, 93%, 94%, 95%, 96%, 97%, 98% or 99% identical with a sequence or sequences selected from SEQ: ID: NO.5, SEQ: ID: NO. 6, SEQ: ID: NO. 7, SEQ: ID: NO. 8 and SEQ: ID: NO. 9.

In a preferred embodiment of the invention, the method envisages putting the at least one isolated sample into contact with at least five portions, preferably with five portions of gliadin as defined above.

In a preferred embodiment of the invention, the method envisages putting the at least one isolated sample into contact with at least five portions or at least five epitopes as defined above.

**Table 1.**

| **Sequence number** | **Sequence** | **Name** |
|---|---|---|
| SEQ ID NO.1 | MKTFLILALLAIVATTARIAVRVPVPQLQPQNPSQ QQPQEQVPLVQQQQFPGQQQPFPPQQPYPQP QPFPSQQPYLQLQPFPQPQLPYPQPQLPYPQPQ LPYPQPQPFRPQQPYPQSQPQYSQPQQPISQQ QQQQQQQQQKQQQQQQQILQQILQQQLIPC RDVVLQQHSIAYGSSQVLQQSTYQLVQQLCCQQ | **Alpha/be ta-gliadin** |
| | LWQIPEQSRCQAIHNVVHAIILHQQQQQQQQ QQPLSQVSFQQPQQQYPSGQGSFQPSQQNPQ AQGSVQPQQLPQFEEIRNLALETLPAMCNVYIPP YCTIAPVGIFGTN | |
| SEQ ID NO.2 | MKTFLIFVLLAMAMNIATAARQLNPSNKELQSPQ QSFSHQQQPFPQQPYPQQPYPSQQPYPSQQPF PTPQPQFPQQSQQPFTQPQQPTPLQPQQPFPQ QPQQPQQPFPQPQQPFPWQPQQPFPQTQQSF PLQPQQPFILQPQQPLPQQPQQPFQQPQQQLS QQPEQTISQQPQQPFPQQPHQPQQPYPQQQPY GSSLTSIGGQ | **Omega-gliadin** |
| SEQ ID NO.3 | MKTLLILTILAMATTIATANMQVDPSGQVQWPQQ QPFPQPQQPFCQQPQRTIPQPHQTFHHQPQQT FPQPQQTYPHQPQQQFPQTQQPQQPFPQPQQ TFPQQPQLPFPQQPQQPFPQPQQPQQPFPQSQ QPQQPFPQPQQQFPQPQQPQQSFPQQQQPAIQ SFLQQQMNPCKNFLLQQCNHVSLVSSLVSIILPR SDCQVMQQQCCQQLAQIPQQLQCAAIHSVAHSII MQQEQQQGVPILRPLFQLAQGLGIIQPQQPAQLE GIRSLVLKTLPTMCNVYVPPDCSTINVPYANIDAG IGGQ | **Gamma-gliadin I** |
| SEQ ID NO.4 | MKTLLILTILAMAITIGTANMQVDPSSQVQWPQQ QPVPQPHQPFSQQPQQTFPQPQQTFPHQPQQ QFPQPQQPQQQFLQPQQPFPQQPQQPYPQQP QQPFPQTQQPQQLFPQSQQPQQQFSQPQQQF PQPQQPQQSFPQQQPPFIQPSLQQQVNPCKNIL LQQCKPASLVSSLWSIIWPQSDCQVMQQQCCQ ELAQIPQQLQCAAIHSVVHSIIMQQQQQQQQQ QQQQGMHILLTLSQQQLGQGTLVQGQGIIQPQ | **Gamma-gliadin II** |
| | QLAQLEAIRSLVLQTLPTMCNVYVPPECSIIRAPF ASIVAGIGGQ | |
| | | |
| SEQ ID NO.5 | QLQPFPQPELPYPQPQP | DQ2.5-glia-α1 a/α2 |
| SEQ ID NO.6 | QPEQPFPQPEQPFPWQP | DQ2.5-glia-ω 1/2 |
| SEQ ID NO.7 | PQQPQQSFPEQEQPA | DQ2.5-glia-γ1 |
| SEQ ID NO.8 | GQGIIQPEQPAQLIR | DQ2.5-glia-γ2 |
| SEQ ID NO.9 | FLQPEQPFPEQPEQPYPEQPEQPFPQ | DQ2.5-glia-γ26 mer |

As is known, the antigenic determinant is the portion of an antigen that is capable of being presented by the HLA-DQ2.5 molecule so that the peptide-HLA complex interacts with the TCR (T-cell receptor) and induces activation of the CD4⁺ T cell. The antigenic determinant is also indicated by the term "epitope".

In one embodiment, the gliadin as defined above is used in the native or deamidated form, partially or completely treated with transglutaminase. Step a) of the method according to the invention is preferably carried out for a period of time between 40 and 48 hours.

In one embodiment of the method of the invention, before step a), the at least one sample isolated from the individual is put into contact with at least one molecule capable of specifically binding CD40. Said molecule capable of specifically binding CD40 is an anti-CD40 antibody that brings about a pre-activation.

Step b) of the method according to the invention comprises detecting the presence of activated T cells in the sample after step a).

In the context of the present invention, the activated T cells are cells of the CD4⁺ subtype which express the two markers OX40 and CD137 on the cell surface.

Preferably, step b) of the method envisages the detection of at least two CD4⁺ T cell activation markers expressed by said T cells.

More preferably, said at least two CD4+ T cell activation markers are OX40 and CD137.

In fact, the presence of such markers demonstrates that the T cells have recently come into contact with at least one portion of gliadin.

In the context of the present description, the activated T cells are CD3⁺/CD4⁺ T cells, which thus express two activation markers, OX40 and CD137.

In a particularly preferred embodiment, the cells detected in the in vitro method are quadruple cells positive for the markers CD3, CD4, OX40 and CD137, i.e. they are CD3⁺/CD4⁺/OX40⁺/CD137⁺ cells.

In one embodiment, the isolated sample is incubated with 4 antibodies that specifically bind to the above-described cell markers. Preferably, the isolated sample is incubated with at least two antibodies that specifically bind to the above-described cell markers, preferably to OX40 and CD137. Preferably, the isolated sample is incubated with at least three antibodies that specifically bind to the above-described cell markers, preferably to CD4, OX40 and CD137.

Preferably, the isolated sample is incubated with at least four antibodies that specifically bind to the above-described cell markers, preferably to CD3, CD4, OX40 and CD137.

Preferably, the antibodies are marked, for example with a fluorophore, an isotope or a quantum dot, to facilitate detection of the cell markers.

In one embodiment, the amount of activated T cells in the sample of the individual is compared with the amount of T cells in a blood sample obtained/isolated from a healthy/normal individual, for example, an individual known not to have coeliac disease or any condition or symptom associated with the disease. In a further embodiment, the amount of activated T cells is compared with the amount of T cells in a blood sample obtained from a subject in remission, or a subject who has not consumed gluten for a period long enough to allow a decrease in the autoimmune response, for example, no consumption of gluten for at least two weeks and preferably at least one month.

In one embodiment, the method is employed to determine the presence of coeliac disease in an individual by comparing between the active CD4+ T cells in a sample isolated from the individual before consuming gluten and a blood sample isolated from the individual after consuming gluten.

In another embodiment, the amount of activated T cells in the sample isolated from the individual is compared to a sample isolated from an individual with coeliac disease.

In other words, the method of the present invention is also employed to assess the progression or regression of coeliac disease in individuals with coeliac disease.

In other words, the test sample, i.e. the sample isolated from an individual suspected to have the disease, is compared with the amount of activated T cells in a "control" sample, i.e. one isolated from an individual without coeliac disease or who has not consumed gluten for a period long enough to enable a decrease in the autoimmune response.

The presence of a higher amount of activated CD4+ T cells, as defined above, in the sample test compared to the control sample is indicative of positivity to coeliac disease.

Advantageously, the in vitro method described thus far further allows for monitoring the remission of the disease resulting from a gluten-free diet, without relying on invasive practices.

In fact, a gluten-free diet, which at present is the only useful therapy, though effective in eliminating the symptoms and restoring intestinal function, poses many restrictions, above all as regards patient compliance with the diet, particularly in the case of teenage patients. In view of this, it is fundamental to monitor the pattern of the disease.

In one embodiment, the detection of a higher amount of activated CD4⁺ T cells detected in step b) compared to an amount of activated CD4⁺ T cells detected in a sample isolated from a healthy individual, i.e. without coeliac disease, indicates the presence of coeliac disease in the individual.

In fact, as shown in the experimental section, following a stimulation with a peptic-tryptic digest of gliadin or with gliadin peptides having the sequences SEQ: ID: NO. 5 - SEQ: ID: NO: 9, one obtains higher percentages of activated T cells in the blood samples isolated from patients with active disease than in patients with a gluten-free diet (GFD) and healthy controls (see example 1 and figure 1 B).

Step b) of detecting active T cells can be carried out with any suitable method known in the art, including a visual count of cells observed under a microscope, or with automated cell counting methods. For example, the cells are counted using a flow cytofluorometer, a Coulter counter, a CASY counter, a haemocytometer or a microscopic image.

Preferably, the cells are distinguished based on their shape, intracellular structures, staining characteristics and the presence of cell markers.

More preferably, the cell markers can be detected with methods that include, by way of example, analysis with immunofluorescent antibodies (IFA), enzyme-linked immuno-culture assay (ELICA), flow cytometry, mass cytometry by time-of-flight (CyTOF) and magnetic cell selection.

In one embodiment, the automated cell count can be carried out with a flow cytometer, a Coulter counter, a CASY counter or with an automated analysis of microscope images. Coulter and CASY counters can be used to measure the volumes and number of cells. Flow cytometry can be used to automatically count and sort cells and detect markers. Furthermore, the microscopic analysis of the cells can be automated. For example, microscope images can be analysed using statistical classification algorithms that automate cell detection and counting. Preferably, step b) is carried out by flow cytometry.

A **second** aspect of the present invention relates to a kit comprising at least one portion of gliadin and at least 2 molecules capable of specifically binding T-cell activation markers, wherein said markers are OX40 and CD137.

Preferably, the kit comprises the at least one portion of gliadin that comprises or consists of at least one sequence selected from SEQ: ID: NO.1-4, preferably at least one sequence selected from: SEQ ID NOs: 5-9. In one embodiment, the kit comprises at least two portions, preferably at least three, more preferably at least four, even more preferably at least five portions of gliadin as described above.

In one embodiment, the kit comprises at least one molecule capable of specifically binding CD40, for example an anti-CD40 antibody.

A **third** aspect of the present invention relates to the use of the kit as defined above to determine the presence of coeliac disease in an individual, preferably according to the method of the invention.

A **fourth** aspect of the present invention relates to a method for monitoring compliance with the therapy for coeliac disease in an individual or preventing the onset of coeliac disease and the symptoms thereof in an individual.

Said method comprises at least one step of detecting the presence of T cells that are reactive against at least one portion of gliadin as described above in detail.

Said method comprises at least steps a) and b) of the method as described above in detail and at least a further step of administering, to said individual, at least one therapy for treating or preventing the onset of coeliac disease and the symptoms thereof and/or at least one step of administering a gluten-free diet.

### EXAMPLES

The present invention will now be illustrated with non-limiting, merely illustrative examples of one of the embodiments of the present invention.

### Example 1 - Stimulation of PBMCs isolated from a patient with gliadin peptides

For the purpose of testing the method according to the present invention, patients with acute coeliac disease, patients in remission on a gluten-free diet and healthy controls without coeliac disease were enrolled after giving informed consent (approval of the ethics committee of University Federico II, N 178/19). In particular, blood samples were isolated from a cohort of 10 patients with active disease (CeD), 10 patients in remission, after following a gluten-free diet (GFD), and 10 healthy controls (CTRL) and the peripheral blood mononuclear cells (PBMCs) were purified.

The cells were immediately frozen in liquid nitrogen and cryopreserved until the experiments were carried out. Subsequently, the cells were plated at a concentration of 1x10⁶ PBMCs/well in 9-well plates in a final volume of 200 µl of RPMI. Before the addition of stimulants, the PBMCs were pretreated with 0.5 µg/mL of anti-CD40 mAb solution for 15 minutes at 37°C. The cells were then stimulated for 24 hours using five highly immunogenic gliadin peptides belonging to the gliadin α-[DQ2.5-glia-*α*1a/*α*2], *ω*-[DQ2.5-glia-*ω*1/2] and *γ*-[DQ2.5-glia-*γ*1, DQ2.5-glia-*γ*2, and *γ-*gliadin 26-mer] families, at a concentration of 10 µg/mL.

The peptides used belong to the proteins (SEQ: ID: NO. 1), (SEQ: ID: NO. 2), (SEQ: ID: NO. 3), (SEQ: ID: NO. 4), or have the sequences (SEQ: ID: NO. 5), (SEQ: ID: NO. 6), (SEQ: ID: NO. 7), (SEQ: ID: NO. 8) and (SEQ: ID: NO. 9) shown in the Table 1:

In parallel, the cells were stimulated with a peptic-tryptic digest of deamidated gliadin (PT-TG gliadin) at 50 µg/mL and with a peptic-tryptic digest of native gliadin (PT gliadin) at 50 µg/mL. The gliadin (PT-TG gliadin) was deamidated by treatment with guinea pig tissue-transglutaminase (t-TG). The peptides (p1-p5) were synthesised in an already deamidated form.

The positive control is produced by stimulating the cells with 2 µg/mL of phytohemagglutinin (PHA, Roche), whereas the unstimulated cells represent the negative control. The immunophenotype was analysed with FACSCanto II (BD Biosciences) and the data processed with DIVA software.

As shown in panel B in Figure 2, no differences were observed following stimulation with the three different antigenic formulations, compared to the medium, in the CTLR and GFD patient groups. In contrast, all the subjects with active coeliac disease show significantly higher percentages of activated T cells (CD3⁺/CD4⁺/OX40⁺/CD137⁺) than the control.

Panel C in Figure 2, on the other hand, shows the percentage of CD3⁺/CD4⁺/OX40⁺/CD137⁺ cells monitored in each subject belonging to the different cohorts, treated with the three stimuli.

The statistical analysis demonstrates a significant activation only for the group of patients with active coeliac disease versus the control subjects and subjects with coeliac disease in remission.

In conclusion, the results demonstrate significantly higher percentages of reactive T cells (CD3⁺/CD4⁺/OX40⁺/CD137⁺) in the blood samples of patients with active disease compared to patients in the GFD group and healthy controls after stimulation both with gliadin and with peptides.

## Claims

1. An in vitro method for determining the presence of coeliac disease in an individual, comprising at least the following steps:
a) putting at least one sample isolated from the individual into contact with gliadin or with at least a portion thereof,
b) detecting the presence of activated T cells in said isolated sample after step a),
wherein a higher amount of activated T cells detected in step b) compared to an amount of activated T cells detected in a sample isolated from an individual without coeliac disease indicates the presence of coeliac disease in the individual,
and wherein step b) comprises the detection of two T-cell activation markers expressed by said T cells.

2. The method according to claim 1, wherein the gliadin is a sequence having a sequence identity of at least 80% with a sequence selected from: SEQ ID NO. 1, SEQ ID NO. 2, SEQ ID NO. 3 and SEQ ID NO. 4 and combinations thereof.

3. The method according to claim 1 or 2, wherein the gliadin is a sequence selected from: SEQ ID NO. 1, SEQ ID NO. 2, SEQ ID NO. 3 and SEQ ID NO. 4 and combinations thereof.

4. The method according to any one of claims 1-3, wherein said two T cell activation markers are OX40 and CD137.

5. The method according to any one of claims 1-4, wherein the T cells are CD3⁺/CD4⁺ lymphocytes.

6. The method according to any one of claims 1-5, wherein said at least one portion of gliadin comprises a sequence at least 80% identical with at least one sequence selected from SEQ: ID: NO.5, SEQ: ID: NO. 6, SEQ: ID: NO. 7, SEQ: ID: NO. 8, and SEQ: ID: NO. 9.

7. The method according to any one of claims 1-6, wherein said at least one portion of gliadin comprises a sequence consisting of at least one sequence selected from SEQ: ID: NO.5, SEQ: ID: NO. 6, SEQ: ID: NO. 7, SEQ: ID: NO. 8, and SEQ: ID: NO. 9.

8. The method according to any one of claims 1-7, wherein step a) comprises putting the at least one sample isolated from the individual into contact with at least two portions of gliadin.

9. The method according to any one of claims 1-8, wherein step a) comprises putting the at least one sample isolated from the individual into contact with at least three portions of the protein.

10. The method according to any one of claims 1-9, wherein step a) comprises putting the at least one sample isolated from the individual into contact with at least four portions of the protein.

11. The method according to any one of claims 1-10, wherein step a) comprises putting the at least one sample isolated from the individual into contact with at least five portions of the protein.

12. The method according to any one of preceding claims, wherein before step a), the at least one sample isolated from the individual is put into contact with at least one molecule capable of specifically binding CD40.

13. A kit comprising at least one portion of gliadin having at least 80% identity with at least one sequence selected from SEQ ID NO.1-4 and at least two molecules capable of specifically binding two T-cell activation markers, wherein said markers are OX40 and CD137.

14. The kit according to claim 13, wherein the at least one portion of gliadin comprises or consists of at least one sequence selected from SEQ: ID: NO. 5, SEQ: ID: NO. 6, SEQ: ID: NO. 7, SEQ ID NO.8 and SEQ: ID: NO. 9.

15. In vitro use of the kit according to claim 13 or 14 to determine the presence of coeliac disease in an individual.
